# FASCICULE DE BREVET EUROPEEN

(11) **EP 1 084 696 B1**
(45) Date de publication et mention de la délivrance du brevet: **10.12.2003**
(21) Numéro de dépôt: 00402375.0
(22) Date de dépôt: 28.08.2000
(51) Int. Cl.: A61K 7/11, A61K 7/06

(54) **Composition cosmétique comprenant au moins un copolymère silicone/acrylate et au moins un agent photoprotecteur**
Kosmetische Zusammensetzung enthaltend mindestens ein Silikon-Acrylat Copolymer und mindestens ein Lichtschutzmittel
Cosmetic composition containing at least a silicone/acrylate copolymer and at least a photoprotectant

(30) Priorité: 16.09.1999 FR 9911593
(43) Date de publication de la demande: 21.03.2001
(60) Demande divisionnaire: 03290733.9
(73) Titulaire: L'OREAL, 75008 Paris (FR)
(72) Inventeur: Dupuis, Christine, 75018 Paris (FR)
(74) Mandataire: Bourdeau, Françoise

(56) Documents cités:
- EP-A- 0 408 311
- EP-A- 0 749 747
- WO-A-99/04750

## Description

L'invention a pour objet une composition cosmétique, comprenant au moins un copolymère silicone/acrylate particulier et au moins un agent photoprotecteur. Elle vise également un procédé cosmétique, en particulier un procédé de fixation et/ou de maintien de la coiffure mettant en oeuvre ladite composition ainsi que l'utilisation de cette composition pour la fabrication d'une formulation cosmétique, destinée notamment au maintien et/ou à la mise en forme de la coiffure.

Selon l'invention, on entend désigner de manière générale par agent photoprotecteur, tout composé ou toute association de composés, connu(s)en soi qui, par des mécanismes, également connus en soi, d'absorption et/ou de réflexion et/ou diffusion du rayonnement UV-A et/ou UV-B, permet d'empêcher, ou du moins limiter, la mise en contact dudit rayonnement avec la surface des cheveux sur laquelle ce ou ces composés ont été appliqués. En d'autres termes, ces composés peuvent être des filtres organiques photoprotecteurs absorbeurs d'UV ou des (nano-)pigments minéraux diffuseurs et/ou réflecteurs d'UV, ainsi que leurs mélanges.

Parmi les produits capillaires, notamment ceux destinés à la mise en forme et/ou au maintien de la coiffure les plus répandus sur le marché de la cosmétique, on distingue des compositions à pulvériser essentiellement constituées d'une solution le plus souvent alcoolique ou aqueuse et d'un ou plusieurs matériaux.

Lorsque les compositions sont destinées à la fixation et/ou au maintien de la coiffure, ces matériaux sont généralement des résines polymères, dont la fonction est de former des soudures entre les cheveux, appelés encore matériaux fixants, en mélange avec divers adjuvants cosmétiques. Ces compositions sont généralement conditionnées soit dans un récipient aérosol approprié mis sous pression à l'aide d'un propulseur, soit dans un flacon pompe.

On connaît également les gels ou les mousses capillaires qui sont généralement appliqués sur les cheveux mouillés avant de faire un brushing ou une mise en plis. Pour mettre en forme et fixer la coiffure, on effectue ensuite un brushing ou un séchage. Ces gels ou mousses peuvent également contenir des résines polymères.

On sait que les radiations lumineuses de longueurs d'onde comprises entre 280 nm et 400 nm altèrent les cheveux en cas d'exposition trop longue d'une personne à ces rayonnements. En particulier, les rayons de longueurs d'onde comprises entre 280 nm et 320 nm, connus sous la dénomination d'UV-B, fragilisent les cheveux et en dégradant ses différents constituants, en particulier la kératine.

Les rayons UV-A, de longueurs d'onde comprises entre 320 nm et 400 nm sont, eux, susceptibles de modifier la couleur naturelle des cheveux, notamment dans le cas de cheveux de couleur claire. Les rayons UV-A provoquent, aussi, une perte d'élasticité des cheveux qui deviennent secs et cassants, surtout au-niveau des pointes.

Il a déjà été proposé des compositions capillaires comprenant des agents photoprotecteurs capables de filtrer les rayons UV-A et UV-B. Toutefois, ces compositions capillaires ne donnent pas encore entière satisfaction, car une trop faible proportion d'agents photoprotecteurs se fixent sur les cheveux. Par conséquent, la protection véritablement obtenue, après application de la composition capillaire sur les cheveux, est loin d'être égale la protection escomptée.

De surcroît, les agents photoprotecteurs qui se sont initialement fixés sur les cheveux n'y restent pas suffisament longtemps pour assurer à la personne une protection convenable et durable. Les cheveux sont donc soumis au rayonnement solaire naturel, c'est-à-dire à un rayonnement contenant des UV-A et UV-B nocifs, peu de temps déjà après l'application de la composition capillaire sur les cheveux.

Il existe donc un besoin de trouver des compositions cosmétiques, notamment pour le maintien et /ou la fixation de la coiffure, qui ne présentent pas l'ensemble des inconvénients indiqués ci-dessus, et qui en particulier fixent bien la coiffure, tout en filtrant suffisamment les rayonnement solaires et en procurant de bonnes propriétés cosmétiques.

De manière surprenante et inattendue, la Demanderesse a découvert que lorsque l'on associe certains copolymères silicone/acrylate avec des agents photoprotecteurs, il est possible d'obtenir des compositions cosmétiques répondant aux exigences exprimées ci-dessus.

L'invention a pour objet une composition cosmétique comprenant, dans un milieu cosmétiquement acceptable, au moins un copolymère silicone/acrylate et au moins un agent photoprotecteur, le copolymère silicone/acrylate étant obtenu par polymérisation radicalaire d'au moins un monomère éthyléniquement insaturé (a) en présence d'au moins un dérivé siliconé (b) comprenant des groupements oxyalkylénés, l'agent photoprotecteur étant choisi parmi les dérivés de l'acide cinnamique et la composition comprenant de 0,2 à 10% d'agent photoprotecteur.

Un autre objet de l'invention concerne un procédé cosmétique capillaire, en particulier un procédé de fixation et/ou de maintien de la coiffure mettant en oeuvre ladite composition.

Encore un autre objet de l'invention concerne l'utilisation de cette composition pour la fabrication d'une formulation cosmétique capillaire, destinée notamment au maintien et/ou à la mise en forme de la coiffure.

Les copolymères silicone/acrylate particulièrement visés par la présente invention sont ceux décrits dans la demande internationale W099/04750. En particulier, on préfère le copolymère commercialisé par BASF sous la dénomination Luviflex Silk. Ce polymère un copolymère greffé acrylate de tertiobutyle/ acide méthacrylique et silicone copolyol.

De préférence, le monomère (a) répond à la formule (Iₐ) : 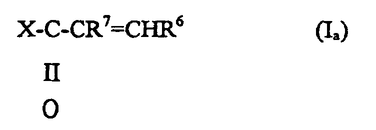 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁴⁺ , alkylammonium, dialkylammonium, trialkylammonium, et tétraalkylammonium ;
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, le 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

En particulier, les monomères (a) de formule (la) sont l'acide acrylique et ses sels, esters ou amides. Les esters peuvent être dérivés d'alkyles linéaires en C₁ à C₄₀, d'alkyles ramifiés en C₃ à C₄₀ ou d'alcools carboxyliques en C₃ à C₄₀, d'alcools polyfonctionnels ayant 2 à 8 hydroxyles, tel que l'éthylène glycol, l'hexylène glycol, le glycérol et le 1,2,6-hexanetriol, d'aminoalcools ou d'éthers d'alcools tels que le méthoxyméthanol et l'éthoxyéthanol ou les polyalkylènes glycols.

Les monomères (a) de formule (la) peuvent également être choisis parmi les N,N-dialkylaminoalkyl acrylates et méthacrylates et N-dialkylaminoalkylacryl- et - méthacrylamides, le groupement amide pouvant être non substitué, N-aklyl- ou N-alkylamino-monosubstitué ou N,N-dialkylamino-disubstitué, les groupes alkyles ou alkylamino étant dérivés d'unités carboxyliques linéaires en C₁ à C₄₀ ou ramifiées en C₃ à C₄₀.

Les monomères (a) de formule (Ia) pouvant être utilisés peuvent aussi être des composés substitués dérivés de l'acide acrylique ou ses sels, esters ou amides. On peut citer par exemple les acides méthacrylique, éthacrylique et 3-cyanoacrylique.

D'autres monomères (a) convenant particulièrement bien sont les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, et les composés hétérocycles substitués par des groupement vinyles ou allyles, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

Comme autre monomère (a), on peut enfin citer les N-vinylimidazoles, les diallylamines, le chlorure de vinylidène, les composés insaturés carbonés comme le styrène et l'isoprène, les dérivés quaternisés par l'épichlorohydrine de l'acide acrylique ou méthacrylique.

On préfère tout particulièrement, comme monomère (a) l'acide acrylique, méthacrylique et éthylacrylique ; le méthyl, éthyl, propyl, n-butyl, isobutyl, t-butyl, 2-éthylhexyl et décyl acrylate; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyéthyl acrylate ; le 2-éthoxyéthyl méthacrylate ; le 2-éthoxyéthyl éthacrylate ; l'hydroxypropyl méthacrylate ; le glycéryl monoacrylate; le glycéryl monométhacrylate; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

Le monomère (a) peut contenir également des atomes de silicium, de fluor ou encore des groupements thio. Les monomères (a) peuvent être neutralisés s'ils contiennent des groupement acides, et ceci avant ou après la polymérisation, totalement ou partiellement, de façon à ajuster la solubilité ou le degré de dispersion dans l'eau au niveau désiré. En tant qu'agent servant pour la neutralisation, on peut utiliser les bases minérales, telles que le carbonate de sodium, les bases oraganiques telles que les aminoalcools, comme les alcanolamines telles que la méthanolamine, le 2-amino-2-méthyl-1-propanol, la triéthanolamine, et les diamines comme la lysine.

Les monomères (a) peuvent être aussi quaternisés lorsqu'ils possèdent-un atome d'azote basique. S'ils possèdent au moins deux doubles liaisons éthyléniques, les monomères (a) peuvent être partiellement réticulés.

Les dérivés siliconés (b) sont notamment les composés connus, sous la dénomination INCI par diméthicone copolyols ou les tensioactifs siliconés. On peut citer ceux commercialisés sous la marque Abil ® par Goldschmidt, Alkasil ® par Rhône-Poulenc, silicone Polyol Copolymer ® par Genesee, Besil ® par Wacker, Silwet ® par OSI ou Dow Corning 190® par Dow Corning. Parmi les monomères (b), on préfère ceux qui présentent la formule I ci-après : 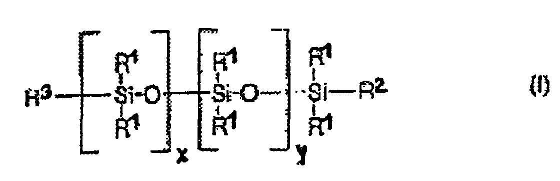 dans laquelle formule (I):
- R² est choisi parmi CH₃ ou le groupement 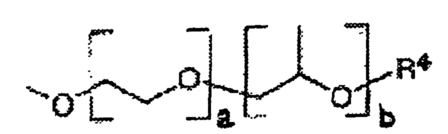
- R³ est choisi parmi CH₃ ou le groupement R²,
- R⁴ est choisi parmi l'hydrogène, CH₃, ou les groupements 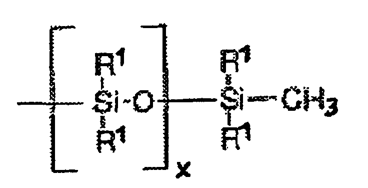 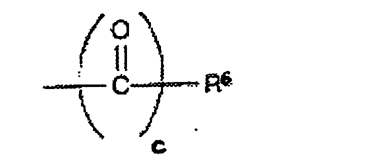
- R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 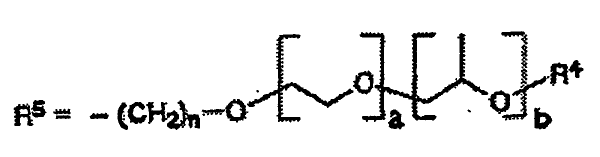
- n est un nombre entier compris entre 1 et 6,
- x et y sont des nombres entiers choisis de telle sorte que le poids moléculaire du polysiloxane soit compris entre 300 et 30 000,
   a et b sont des nombres entiers compris entre 0 et 50, et
- c est 0 ou 1.

De préférence, R¹ est choisi parmi les groupements méthyl, éthyl, propyl, butyl, isobutyl, pentyl, isopentyl; hexyl, octyl, décyl, dodécyl and octadécyl, les radicaux cycloaliphatiques, en particulier les groupements cyclohexyls, les groupements aromatiques, en particulier les groupements phényls ou naphthyls, les mélanges de radicaux aromatiques et aliphatiques, tels que le benzyl et le phenyléthyl, et aussi le tolyl and le xylyl.

On préfère les radicaux R⁴ ayant pour formule -(CO)c-R⁶, R⁶ étant un alkyl, cycloalkyl or aryl ayant 1 à 40 atomes de carbone qui peut contenir des groupements supplémentaires tels que NH₂, COOH et/ou SO₃H.

On préfère les radicaux R⁶ pour lesquels c=0 qui sont des phosphates ou des sulfates.

Les dérivés siliconés (b) particulièrement préférés sont ceux présentant la formule générale suivante :

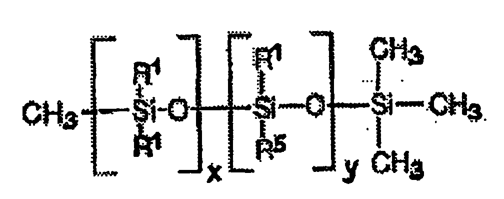

La proportion relative de dérivé (b) dans le copolymère est généralement de 0,1 à 50, et de préférence de 1 à 20 % en poids.

On préfère les copolymères silicone/acrylate solubles dans l'eau ou ceux dont la dispersibilité dans l'eau est telle que dans un mélange eau/éthanol dosé à 50 :50 en volume, ils sont solubles dans une proportion supérieure à 0,1 g/l, et de préférence supérieure à 0,2 g/l.

La composition comprend avantageusement, en pourcentage relatif en poids de la composition, de 0,1 à 20 % de ce copolymère silicone/acrylate, et de préférence, de 0,5 à 10%.

La composition comprend avantageusement, en pourcentage relatif en poids de la composition, d'agent photoprotecteur de 0,2 à 10 %.

L'agent photoprotecteur est choisi parmi, les dérivés de l'acide cinnamique tels que le 4-méthoxy cinnamate d'isopentyle, le 4-méthoxy cinnamate de 2-éthylhexyle, le diisopropyl cinnamate de méthyle, le 4-méthoxy cinnamate d'isoamyle, le 4-méthoxy cinnamate de diéthanolamine.

Parmi les dérivés de l'acide cinnamique mentionnés ci-dessus, on préfère tout particulièrement, selon la présente invention, mettre en oeuvre le p-méthoxycinnamate de 2-éthylhexyle, vendu notamment sous le nom commercial « PARSOL MCX » par la Société GIVAUDAN.

Les compositions cosmétiques selon l'invention peuvent encore contenir, en tant qu'agent photoprotecteurs, des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Le milieu cosmétiquement acceptable est, de préférence, constitué par de l'eau ou un ou plusieurs solvants cosmétiquement acceptables tels que des alcools ou des mélanges eau-solvant(s), ces solvants étant de préférence des alcools en C₁-C₄.

Parmi ces alcools, on peut citer l'éthanol, l'isopropanol. L'éthanol est particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les polymères autres que ceux de l'invention, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Ces compositions peuvent être conditionnées sous diverses formes, notamment dans des flacons pompes ou dans des récipients aérosols, afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux. Les compositions conformes à l'invention peuvent aussi se présenter sous la forme de crèmes, de gels, d'émulsions, de lotions ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.

Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.

Les compositions conformes à l'invention sont particulièrement adaptés pour des cheveux secs ou humides, en tant que produits de coiffage.

Les compositions conformes à l'inventions peuvent être appliquées sur la peau, les ongles, les lèvres, les cheveux, les sourcils et les cils.

## Revendications

1. Composition cosmétique comprenant, dans un milieu cosmétiquement acceptable, au moins un copolymère silicone/acrylate et au moins un agent photoprotecteur, **caractérisée par le fait que** le copolymère silicone/acrylate est obtenu par polymérisation radicalaire d'au moins un monomère éthyléniquement insaturé (a) en présence d'au moins un dérivé siliconé (b) comprenant des groupements oxyalkylénés, l'agent photoprotecteur étant choisi parmi les dérivés de l'acide cinnamique et la composition comprenant de 0,2 à 10 % d'agent photoprotecteur.

2. Composition selon la revendication 1, **caractérisée par le fait que** le monomère (a) répond à la formule (Iₐ) : 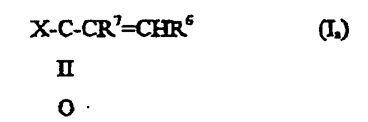 dans laquelle :
- X est choisi dans le groupe comprenant OH, OM, OR⁸, NH₂, NHR⁸, N(R⁸)₂ ;
- M est un cation choisi parmi Na⁺, K⁺, Mg⁺⁺, NH ⁺⁺ , alkylammonium, dialkylammonium, trialkylammonium, et tétraalkylammonium ,
- les radicaux R⁸ peuvent être identiques ou différents et sont choisis dans le groupe comprenant l'hydrogène, les groupements alkyls linéaires ou ramifiés en C₁ à C₄₀, les groupements alkyls en C₁ à C₄₀ mono- ou polyhydroxylés, éventuellement substitués par un ou plusieurs groupement(s) alcoxy, amino ou carboxy, les groupements polyéthers hydroxylés, les groupements N,N-diméthylaminoéthyl, 2-hydroxyéthyl, 2-méthoxyéthyl, 2-éthoxyéthyl, hydroxypropyl, méthoxypropyl et éthoxypropyl ;
- R⁷ et R⁶ sont choisis indépendamment l'un de l'autre dans le groupe comprenant l'hydrogène, les groupements alkyls linéaires ou ramifiés en C₁ à C₈, méthoxy, éthoxy, 2-hydroxyéthoxy, 2-méthoxyéthoxy et 2-éthoxyéthyl, les groupements CN, COOH et COOM.

3. Composition cosmétique selon la revendication 1, **caractérisée par le fait que** le dérivé siliconé (b) répond à la formule I ci-après : 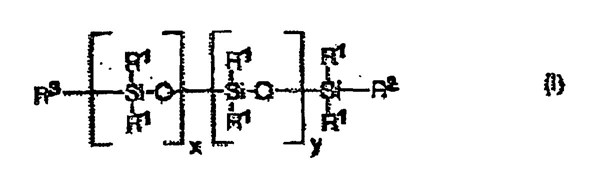 dans laquelle formule (I):
- R² est choisi parmi CH₃ ou le groupement 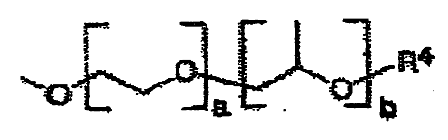
- R³ est choisi parmi CH₃ ou le groupement R²,
- R⁴ est choisi parmi l'hydrogène, CH₃, ou les groupements 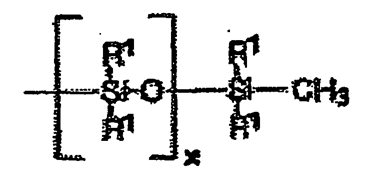 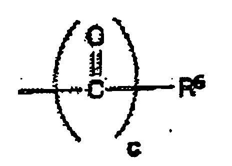 R⁶ est un groupement organique en C₁ à C₄₀ pouvant contenir des groupements amino, carboxyles ou sulfonates, et si c=0, R₆ est l'anion d'un acide inorganique ;
- les radicaux R¹ peuvent être identiques ou différents et sont choisis parmi les hydrocarbures aliphatiques en C₁ à C₂₀, les hydrocarbures aliphatiques ou cycloaliphatiques en C₃ à C₂₀, et les groupements R⁵ répondant à la formule ci-après : 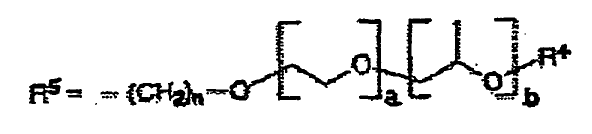
- n est un nombre entier compris entre 1 et 6,
- x et y sont des nombres entiers choisis de telle sorte que le poids moléculaire du polysiloxane soit compris entre 300 et 30 000,
- a et b sont des nombres entiers compris entre 0 et 50, et
- c est 0 ou 1.

4. Composition selon la revendication 1, **caractérisée par le fait que** le monomère (a) est choisi parmi les esters de vinyle et d'allyle en C₁ à C₄₀, les acides carboxyliques, linéaires en C₃ à C₄₀, ou carboxycycliques en C₃ à C₄₀, les halides de vinyle ou d'allyle, les vinyllactames, de préférence la vinylpyrrolidone et le vinylcaprolactame, les composés hétérocycles substitués par des groupement vinyls ou allyls, de préférence la vinylpyridine, la vinyloxazoline et l'allylpyridine, les N-vinylimidazoles, les diallylamines, le chlorure de vinylidène, les composés insaturés carbonés, comme le styrène ou l'isoprène, les dérivés quaternisés par l'épichlorhydrine de l'acide acrylique ou méthacrylique.

5. Composition selon la revendication 1, **caractérisée par le fait que** le monomère (a) est choisi dans le groupe comprenant l'acide acrylique, méthacrylique et éthylacrylique ; le méthyl, éthyl, propyl, n-butyl, isobutyl, t-butyl, 2-éthylhexyl et décyl acrylate ; le méthyl, éthyl, propyl, n-butyl, isobutyl, t-butyl, 2-éthylhexyl et décyl méthacrylate ; le méthyl, éthyl, propyl, n-butyl, isobutyl, t-butyl, 2-éthylhexyl et décyl éthacrylate ; le 2,3-dihydroxypropyl acrylate ; le 2,3-dihydroxypropyl méthacrylate ; le 2-dihydroxyéthyl acrylate ; l'hydroxypropyl acrylate ; le 2-hydroxyéthyl méthacrylate ; le 2-hydroxyéthyl éthacrylate ; le 2-méthoxyethyl acrylate ; le 2-éthoxyéthyl méthacrylate ; le 2-éthoxyéthyl éthacrylate ; l'hydroxypropyl méthacrylate ; le glycéryl monoacrylate ; le glycéryl monométhacrylate ; les polyalkylènes glycols( méth-) acrylate, les acides sulfoniques insaturés, l'acrylamide, la méthacrylamide, l'éthacrylamide, la N,N-diméthylacrylamide, la N-éthylacrylamide, la N-éthylméthacrylamide, 1-vinylimidazole, N,N-diméthylaminoéthyl(méth-)acrylate, l'acide maléique, l'acide fumarique, l'anhydride maléique et ses monoesters, l'acide crotonique, l'acide itaconique, les éthers de vinyle, le vinylformamide, la vinylamine, la vinylpyridine, la vinylimidazole, le vinylfurane, le styrène, le sulfonate de styrène, l'alcool allylique et leurs mélanges.

6. Composition selon l'une quelconque des revendications précédentes, **caractérisée par le fait que** les dérivés siliconés (b) sont choisis parmi les diméthicone copolyols ou les tensioactifs siliconés.

7. Composition selon l'une quelconque des revendications précédentes, **caractérisée par le fait qu'**elle comprend, en pourcentage relatif en poids, de 0,1 à 20 % de copolymère silicone/acrylate, et plus préférentiellement de 0,5 à 10 % de ce copolymère.

8. Composition selon l'une quelconque des revendications précédentes, **caractérisée par le fait qu'**elle contient en outre au moins un additif choisi parmi les tensioactifs anioniques, canoniques, non ioniques ou amphotères, les parfums, les conservateurs, les protéines, les vitamines, les polymères autres que ceux de l'invention, les huiles végétales, minérales ou synthétiques, les filtres.

9. Procédé de maintien ou de mise en forme de la coiffure, **caractérisé par le fait qu'**il comprend la mise en oeuvre d'une composition conforme à l'une quelconque des revendications 1 à 8.

10. Utilisation d'une composition selon l'une quelconque des revendications 1 à 8, pour la fabrication d'un produit cosmétique, en particulier capillaire.

11. Utilisation selon l'une quelconque des revendications 1 à 8 pour la peau, les cheveux, les sourcils et les cils.

## Patentansprüche

1. Kosmetische Zusammensetzung, die in einem kosmetisch akzeptablen Medium mindestens ein Silicon/Acrylat-Copolymer und mindestens ein Lichtschutzmittel enthält, **dadurch gekennzeichnet, dass** das Silicon/Acrylat-Copolymer durch radikalische Polymerisation mindestens eines ethylenisch ungesättigten Monomers (a) in Gegenwart mindestens eines Siliconderivats (b), das Oxyalkylen-Gruppen enthält, erhalten wird, wobei das Lichtschutzmittel unter den Zimtsäurederivaten ausgewählt ist und wobei die Zusammensetzung 0,2 bis 10 % Lichtschutzmittel enthält.

2. Zusammensetzung nach Anspruch 1, **dadurch gekennzeichnet, dass** das Monomer (a) der Formel (Iₐ) 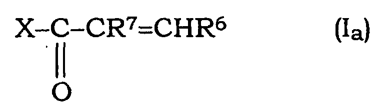 entspricht, in der:
- X unter OH, OM, OR⁸, NH₂, NHR⁸, N(R⁸)₂ ausgewählt ist;
- M ein Kation ist, das unter Na⁺, K⁺, Mg⁺⁺, NH₄⁺, Alkylammonium, Dialkylammonium, Trialkylammonium und Tetraalkylammonium ausgewählt ist;
- die Reste R⁸ identisch oder verschieden sein können und unter Wasserstoff, geradkettigen oder verzweigten C₁₋₄₀-Alkylgruppen, C₁₋₄₀-Mono- oder C₁₋₄₀-Polyhydroxyalkylgruppen, die gegebenenfalls mit einer oder mehreren Alkoxy-, Amino- oder Carboxygruppen substituiert sind, hydroxylierten Polyethergruppen, N,N-Dimethylaminoethyl, 2-Hydroxyethyl, 2-Methoxyethyl, 2-Ethoxyethyl, Hydroxypropyl, Methoxypropyl und Ethoxypropyl ausgewählt sind;
- R⁷ und R⁶ unabhängig voneinander unter Wasserstoff, geradkettigen oder verzweigten C₁₋₈-Alkylgruppen, Methoxy, Ethoxy, 2-Hydroxyethoxy, 2-Methoxyethoxy und 2-Ethoxyethyl, den Gruppen CN, COOH und COOM ausgewählt sind.

3. Kosmetische Zusammensetzung nach Anspruch 1, **dadurch gekennzeichnet, dass** das Siliconderivat (b) der folgenden Formel I 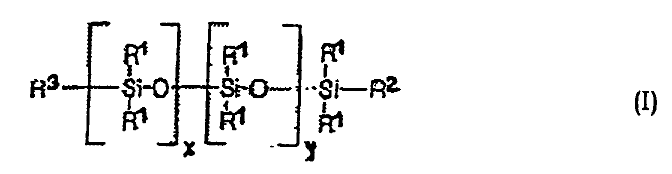 entspricht, wobei
- R² unter CH₃ und der Gruppe 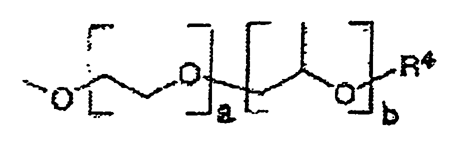 ausgewählt ist,
- R³ unter CH₃ und der Gruppe R² ausgewählt ist,
- R⁴ unter Wasserstoff, CH₃ und den Gruppen 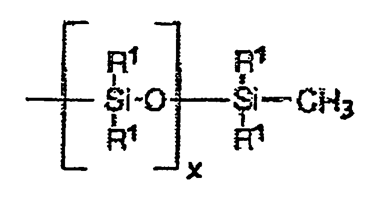 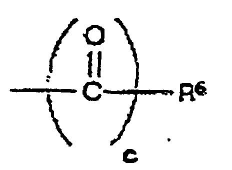 ausgewählt ist,
- R⁶ eine organische C₁₋₄₀-Gruppe ist, die Amino-, Carboxy- oder Sulfonatgruppen enthalten kann, wobei R⁶ das Anion einer anorganischen Säure ist, wenn c gleich Null ist;
- die Reste R¹ gleich oder verschieden sein können und unter den aliphatischen C₁₋₂₀-Kohlenwasserstoffen, den aliphatischen oder cycloaliphatischen C₃₋₂₀-Kohlenwasserstoffen ausgewählt sind, wobei die Gruppen R⁵ der folgenden Formel 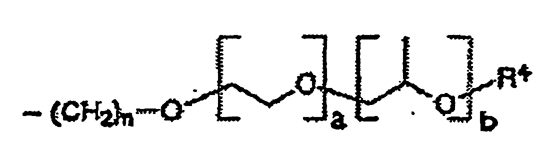 entsprechen,
- n eine ganze Zahl ist, die im Bereich von 1 bis 6 liegt,
- x und y ganze Zahlen sind, die so ausgewählt sind, dass das Molekulargewicht des Polysiloxans im Bereich von 300 bis 30000 liegt,
- a und b ganze Zahlen sind, die im Bereich von 0 bis 50 liegen, und
- c die Zahl 0 oder 1 ist.

4. Zusammensetzung nach Anspruch 1, **dadurch gekennzeichnet, dass** das Monomer (a) ausgewählt ist unter: den C₁₋₄₀-Vinylestern und C₁₋₄₀-Allylestern, den geradkettigen C₃₋₄₀-Carbonsäuren, den carbocyclischen C₃₋₄₀-Carbonsäuren, den Vinyl- oder Allylhalogeniden, den Vinyllactamen; vorzugsweise Vinylpyrrolidon und Vinylcaprolactam, den heterocyclischen Verbindungen, die mit Vinyl- oder Allylgruppen substituiert sind, vorzugsweise Vinylpyridin, Vinyloxazolin und Allylpyridin, den N-Vinylimidazolen, den Diallylaminen, Vinylidenchlorid, den ungesättigten Kohlenstoffverbindungen, wie Styrol oder Isopren, den mit Epichlorhydrin quaternisierten Derivaten von Acrylsäure oder Methacrylsäure.

5. Zusammensetzung nach Anspruch 1, **dadurch gekennzeichnet, dass** das Monomer (a) ausgewählt ist unter Acrylsäure, Methacrylsäure und Ethylacrylsäure; Methyl-, Ethyl-, Propyl-, n-Butyl-, Isobutyl-, tert.-Butyl-, 2-Ethylhexyl- und Decylacrylat; Methyl-, Ethyl-, Propyl-, n-Butyl-, Isobutyl-, tert.-Butyl-, Ethylhexyl- und Decylmethacrylat; Methyl-, Ethyl-, Propyl-, n-Butyl-, Isobutyl-, tert.-Butyl-, Ethylhexylund Decylethacrylat;2,3-Dihydroxypropylacrylat; 2,3-Dihydroxypropylmethacrylat; 2-Dihydroxyethylacryat; Hydroxypropylacrylat; 2-Hyroxyethylmethacrylat; 2-Hydroxyethylethacrylat; 2-Methoxyethylacrylat; 2-Ethoxyethylmethacrylat; 2-Ethoxyethylethacrylat; Hydroxypropylmethacrylat; Glycerinmonoacrylat; Glycerinmonomethacrylat, den Polyalkylenglykol(meth)acrylaten, den ungesättigten Sulfonsäuren, Acrylamid, Methacrylamid, Ethacrylamid, N,N-Dimethylacrylamid, N-Ethylacrylamid, N-Ethylmethacrylamid, 1-Vinylimidazol, N,N-Dimethylaminoethyl(meth)acrylat, Maleinsäure, Fumarsäure, Maleinsäureanhydrid und deren Monoestern, Crotonsäure, Itaconsäure, den Vinylethern, Vinylformamid, Vinylamin, Vinylpyridin, Vinylimidazol, Vinylfuran, Styrol, Styrolsulfonat, Allylalkohol und den Gemischen dieser Verbindungen.

6. Zusammensetzung nach einem der vorhergehenden Ansprüche, **dadurch gekennzeichnet, dass** die Siliconderivate (b) unter den Dimeticoncopolyolen und den siliconhaltigen grenzflächenaktiven Stoffen ausgewählt sind.

7. Zusammensetzung nach einem der vorhergehenden Ansprüche, **dadurch gekennzeichnet, dass** sie, angegeben in Gewichtsprozent, 0,1 bis 20 % Silicon/Acrylat-Copolymer und vorzugsweise 0,5 bis 10 % dieses Copolymers enthält.

8. Zusammensetzung nach einem der vorhergehenden Ansprüche, **dadurch gekennzeichnet, dass** sie außerdem mindestens einen Zusatzstoff enthält, der unter den anionischen, kationischen, nichtionischen oder amphoteren grenzflächenaktiven Stoffen, den Parfüms, den Konservierungsmitteln, den Proteinen, den Vitaminen, den Polymeren, die von den Polymeren der Erfindung verschieden sind, den pflanzlichen, mineralischen oder synthetischen Ölen, den Filtern ausgewählt ist.

9. Verfahren zur Erhaltung oder Gestaltung der Frisur, **dadurch gekennzeichnet, dass** es die Anwendung einer Zusammensetzung nach einem der Ansprüche 1 bis 8 umfaßt.

10. Verwendung einer Zusammensetzung nach einem der Ansprüche 1 bis 8 für die Herstellung eines kosmetischen Produkts, insbesondere eines Produkts zur Haarbehandlung.

11. Verwendung nach einem der Ansprüche 1 bis 8 für die Haut, die Haare, die Wimpern und die Augenbrauen.

## Claims

1. Cosmetic composition comprising, in a cosmetically acceptable medium, at least one silicone/acrylate copolymer and at least one photoprotective agent, **characterized in that** the silicone/acrylate copolymer is obtained by radical-mediated polymerization of at least one ethylenically unsaturated monomer (a) in the presence of at least one silicone derivative (b) comprising oxyalkylene groups, the photoprotective agent being chosen from cinnamic acid derivatives and the composition comprising from 0.2 to 10% of photoprotective agent.

2. Composition according to Claim 1, **characterized in that** the monomer (a) corresponds to formula (Iₐ): 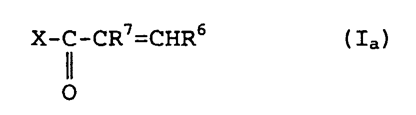 in which:
- X is chosen from the group comprising OH, OM, OR⁸, NH₂, NHR⁸ and N(R⁸)₂;
- M is a cation chosen from Na⁺, K⁺, Mg⁺⁺, NH⁴⁺, alkylammonium, dialkylammonium, trialkylammonium and tetraalkylammonium;
- the radicals R⁸ may be identical or different and are chosen from the group comprising hydrogen, linear or branched C₁ to C₄₀ alkyl groups, mono- or polyhydroxylated C₁ to C₄₀ alkyl groups, optionally substituted with one or more alkoxy, amino or carboxyl groups, hydroxylated polyethers, and N,N-dimethylaminoethyl, 2-hydroxyethyl, 2-methoxyethyl, 2-ethoxyethyl, hydroxypropyl, methoxypropyl and ethoxypropyl groups;
- R⁷ and R⁶ are chosen, independently of each other, from the group comprising hydrogen, linear or branched C₁ to C₈ alkyl groups, methoxy, ethoxy, 2-hydroxyethoxy, 2-methoxyethoxy and 2-ethoxyethyl groups, and CN, COOH and COOM groups.

3. Cosmetic composition according to Claim 1, **characterized in that** the silicone derivative (b) corresponds to the formula (I) below: 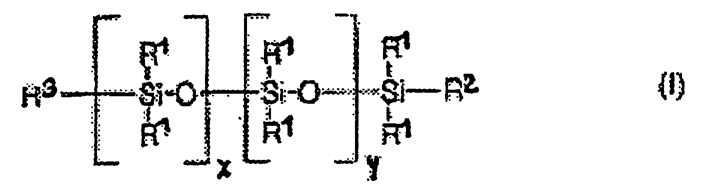 in which formula (I):
- R² is chosen from CH₃ and the group 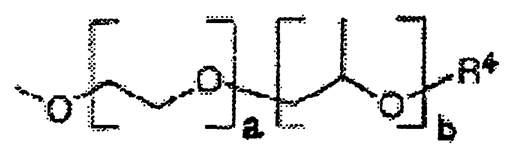
- R³ is chosen from CH₃ and the group R²,
- R⁴ is chosen from hydrogen, CH₃ and the groups 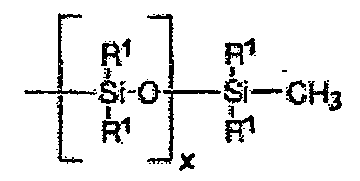 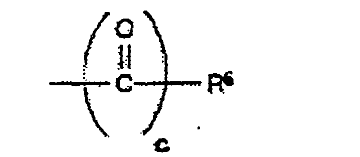
- R⁶ is an organic C₁ to C₄₀ group which can contain amino, carboxyl or sulphonate groups, and if c=0, R₆ is the anion of an inorganic acid;
- the radicals R¹ may be identical or different and are chosen from C₁ to C₂₀ aliphatic hydrocarbons, C₃ to C₂₀ aliphatic or cycloaliphatic hydrocarbons, and the groups R⁵ corresponding to the formula below: 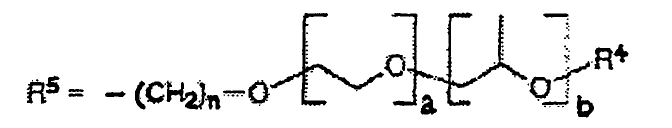
- n is an integer between 1 and 6,
- x and y are integers chosen such that the molecular weight of the polysiloxane is between 300 and 30,000,
- a and b are integers between 0 and 50, and
- c is 0 or 1.

4. Composition according to Claim 1, **characterized in that** the monomer (a) is chosen from C₁ to C₄₀ vinyl and allyl esters, linear C₃ to C₄₀ carboxylic acids or C₃ to C₄₀ carboxycyclic acids, vinyl or allyl halides, vinyllactams, preferably vinylpyrrolidone and vinylcaprolactam, heterocyclic compounds substituted with vinyl or allyl groups, preferably vinylpyridine, vinyloxazoline and allylpyridine, N-vinylimidazoles, diallylamines, vinylidene chloride, carbon-based unsaturated compounds, such as styrene or isoprene, and acrylic or methacrylic acid derivatives quaternized with epichlorohydrin.

5. Composition according to Claim 1, **characterized in that** the monomer (a) is chosen from the group comprising acrylic, methacrylic and ethacrylic acid; methyl, ethyl, propyl, n-butyl, isobutyl, t-butyl, 2-ethylhexyl and decyl acrylate; methyl, ethyl, propyl, n-butyl, isobutyl, t-butyl, 2-ethylhexyl and decyl methacrylate; methyl, ethyl, propyl, n-butyl, isobutyl, t-butyl, 2-ethylhexyl and decyl ethacrylate; 2,3-dihydroxypropyl acrylate; 2,3-dihydroxypropyl methacrylate; 2-dihydroxyethyl acrylate; hydroxypropyl acrylate; 2-hydroxyethyl methacrylate; 2-hydroxyethyl ethacrylate; 2-methoxyethyl acrylate; 2-ethoxyethyl methacrylate; 2-ethoxyethyl ethacrylate; hydroxypropyl methacrylate; glyceryl monoacrylate; glyceryl monomethacrylate; polyalkylene glycol (meth)acrylates, unsaturated sulphonic acids, acrylamide, methacrylamide, ethacrylamide, N,N-dimethylacrylamide, N-ethylacrylamide, N-ethylmethacrylamide, 1-vinylimidazole, N,N-dimethylaminoethyl (meth)acrylate, maleic acid, fumaric acid, maleic anhydride and its monoesters, crotonic acid, itaconic acid, vinyl ethers, vinylformamide, vinylamine, vinylpyridine, vinylimidazole, vinylfuran, styrene, styryl sulphonate and allyl alcohol, and mixtures thereof.

6. Composition according to any one of the preceding claims, **characterized in that** the silicone derivatives (b) are chosen from dimethicone copolyols and silicone surfactants.

7. Composition according to any one of the preceding claims, **characterized in that** it comprises, as a relative percentage by weight, from 0.1% to 20% of silicone/acrylate copolymer, and more preferably from 0.5% to 10% of this copolymer.

8. Composition according to any one of the preceding claims, **characterized in that** it also contains at least one additive chosen from anionic, cationic, nonionic and amphoteric surfactants, fragrances, preserving agents, proteins, vitamins, polymers other than those of the invention, plant, mineral or synthetic oils, and filters.

9. Process for holding or shaping the hairstyle, **characterized in that** it uses a composition in accordance with any one of Claims 1 to 8.

10. Use of a composition according to any one of Claims 1 to 8, for the manufacture of a cosmetic product, in particular a hair product.

11. Use according to any one of Claims 1 to 8, for the skin, the hair, the eyebrows and the eyelashes.
